# EUROPEAN PATENT APPLICATION

(11) **EP 3 111 827 A1**
(43) Date of publication of application: **04.01.2017**
(21) Application number: 15002835.5
(22) Date of filing: 02.10.2015
(51) Int. Cl.: A61B 1/267, A61B 1/00

(54) **VIDEO LARYNGOSCOPE WITH EASY HANDLING FUNCTION**

(30) Priority: 01.07.2015 KR 20150094026
(71) Applicant: Ace Medical Co., Ltd, Gyeonggi-Do 412-520 (KR)
(72) Inventor: LEE, Jong- Woo, 136-060 Seoul (KR)
(74) Representative: Hering, Hartmut

(57) **Abstract**

The present invention relates to a video laryngoscope. More particularly, the present invention relates to a video laryngoscope, in which a guide space having a wide rear end and a narrow front end is defined at a front end of the video laryngoscope by a guide wing and a soft wing, so that a tube can be easily inserted into the guide space and can be easily handled.

## Description

### BACKGROUND OF THE INVENTION

### 1. Field of the Invention

The present invention relates to a video laryngoscope. More particularly, the present invention relates to a video laryngoscope, in which a guide space having a wide rear end and a narrow front end is defined at a front end of the video laryngoscope by a guide wing and a soft wing, so that a tube can be easily inserted into the guide space and can be easily handled.

### 2. Description of the Related Art

In general, a laryngoscope is a medical device used for endotracheal intubation to maintain the respiration tract of a person. The laryngoscope ensures the free airway of a patient for intubation of a tube.

For reference, such a laryngoscope is classified into a mackintosh laryngoscope and a video laryngoscope having a camera mounted on a blade of the laryngoscope.

The mackintosh laryngoscope includes an elongate member having a curved front end. The mackintosh laryngoscope is inserted through an oral cavity of an unconscious patient and ensures the free airway of the patient by using a curved portion formed at a lateral side of the front end for intubation of the tube.

However, the mackintosh laryngoscope is of ten manipulated depending on the operator's experience and eyes, so intubation of the tube may be delayed or failed in case of novices.

In contrast, in case of the video laryngoscope, an image sensor and a lens are installed on a front end of a blade for free airway and an image photographed by the image sensor is displayed on a display device, so that operators can perform intubation of the tube while checking the free airway of the patient displayed on the display device.

For this reason, recently, the video laryngoscope is increasingly used more than the mackintosh laryngoscope due to the convenience of use thereof.

However, since the size of the video laryngoscope is larger than the size of the mackintosh laryngoscope, the volume of the free airway may become small. In particular, the video laryngoscope creates a great amount of foreign substances, so intubation of the tube and the handling work may be difficult.

In addition, since the display device is fixed to one side of a grip part of the video laryngoscope, the display device fixed to the grip part is moved when the operator manipulates the video laryngoscope by using the grip part, so it is necessary to fix the view point of the operator.

As related arts, there are Korean registered patent publication Nos. 10-1245436 (registration date: March 13, 2013) and 10-1245474 (registration date: March 13, 2013).

### SUMMARY OF THE INVENTION

An object of the present invention is to provide a video laryngoscope, which facilitates intubation of a tube and handling work when ensuring free airway by using the video laryngoscope, does not reuse a blade fitted to a front end, and does not require to change a sight of an operator according to a position of the video laryngoscope being handled.

In order to accomplish the above object, the present invention provides a video laryngoscope which includes a grip part provided therein with a body, a front end part that forms a curved frame protruding from a lower end of the grip part in which an image sensor and a lens are installed on an end of the frame, and a blade fitted to the front end part.

The blade includes a top surface curved downward with a curvature identical to a curvature of the front end part, a bottom surface curved downward to an extent of a middle part of the bottom surface with a curvature identical to the curvature of the front end part, and first and second lateral sides to connect the top and bottom surfaces with each other.

A partition wall protrudes upward from the top surface with a curvature less than the curvature of the front end part.

A guide wing protrudes to one side from an upper portion of the partition wall while being connected to the front end part to form a guide space below the guide wing.

A slot having a curvature identical to the curvature of the front end part is formed at one side of the bottom surface and a soft wing is detachably installed in the slot.

A tube is allowed to be readily inserted into the guide space formed between the guide wing and the soft wing such that the tube is easily handled.

In addition, according to the present invention, the video laryngoscope includes a display device detachably assembled to an upper end of the grip part. The display device is wirelessly connected to the body of the grip part and an image photographed by the front end part is displayed on the display device.

According to the present invention, the partition wall having the curvature less than the curvature of the front end part is formed at an upper end of the front end part. In addition, the guide wing extending laterally to the front end part is provided above the partition wall and the soft wing is detachably attached to one side end of the bottom surface.

Thus, the tube can be readily inserted into the guide space formed between the guide wing and the soft wing such that the tube can be easily handled.

In addition, according to the present invention, the display device is detachably mounted on an upper end of the grip part, so it is not necessary for an operator to change the view point in order to check a larynx, so that intubation of a tube can be readily achieved.

### BRIEF DESCRIPTION OF THE DRAWINGS

FIG. 1 is a perspective view illustrating a video laryngoscope according an embodiment of the present invention.
FIG. 2 is an exploded perspective view illustrating a video laryngoscope according an embodiment of the present invention.
FIG. 3 is a right side view of a blade shown in FIG. 2.
FIG. 3a is an exploded perspective view of a blade shown in FIG. 2.
FIGS. 4 and 4a are perspective and left side views of a blade, respectively, taken from the left side of blade.
FIG. 5 is a side view showing states before and after a blade shown in FIG. 2 is mounted.
FIG. 6 is an exploded perspective view of a coupling part for coupling a display device to a grip part.
FIG. 7 is a perspective view showing a state in which a display device is rotatably coupled to a grip part.

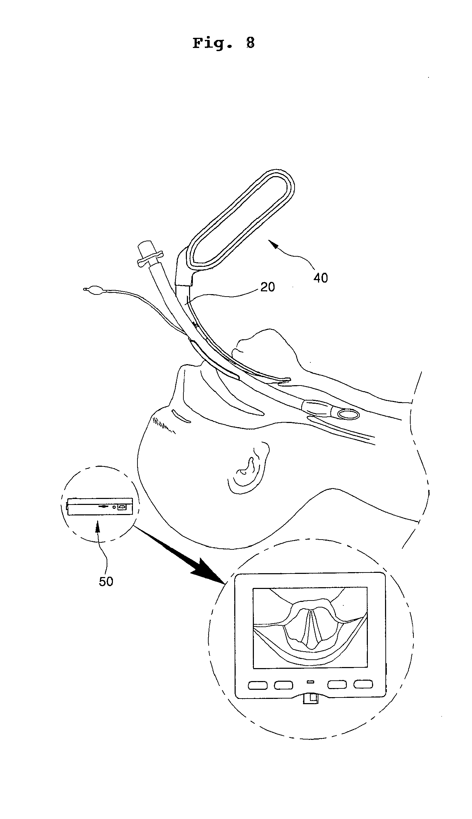

FIG. 8 is a view showing intubation of a tube into an airway according to an embodiment of the present invention.

### DETAILED DESCRIPTION OF THE INVENTION

Hereinafter, embodiments of the present invention will be described in detail with reference to the accompanying drawings.

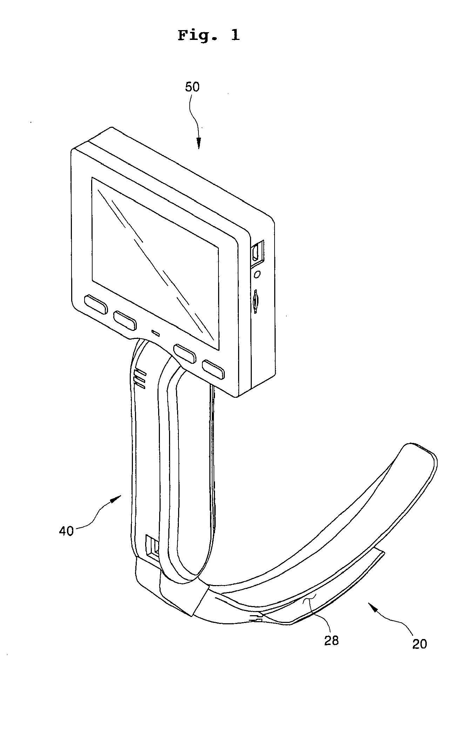

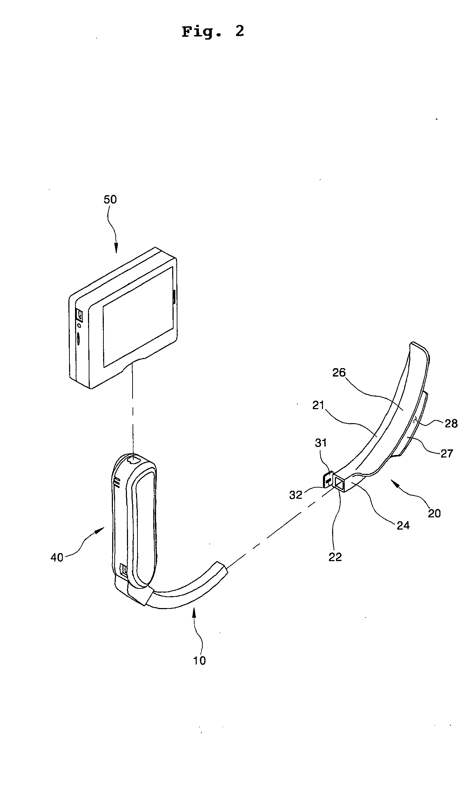

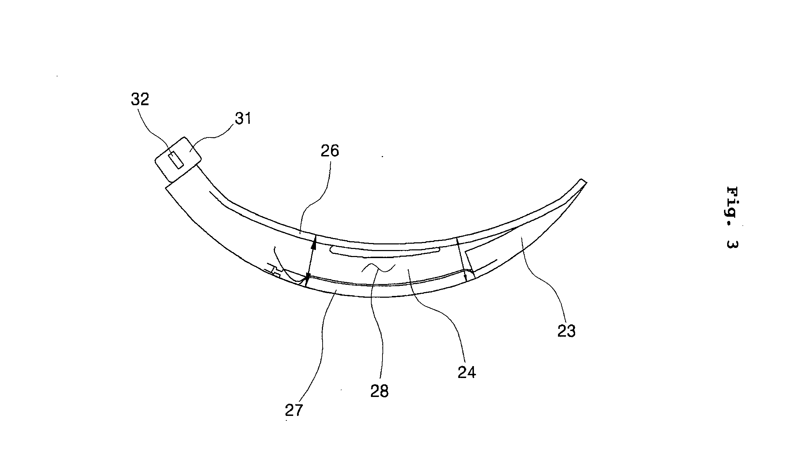

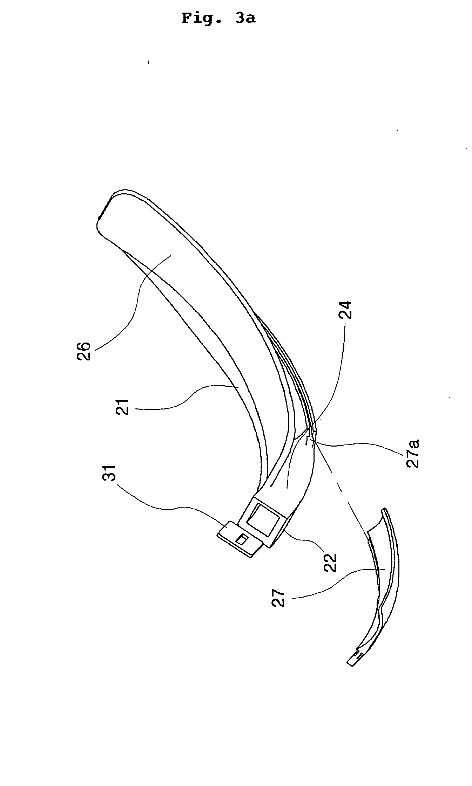

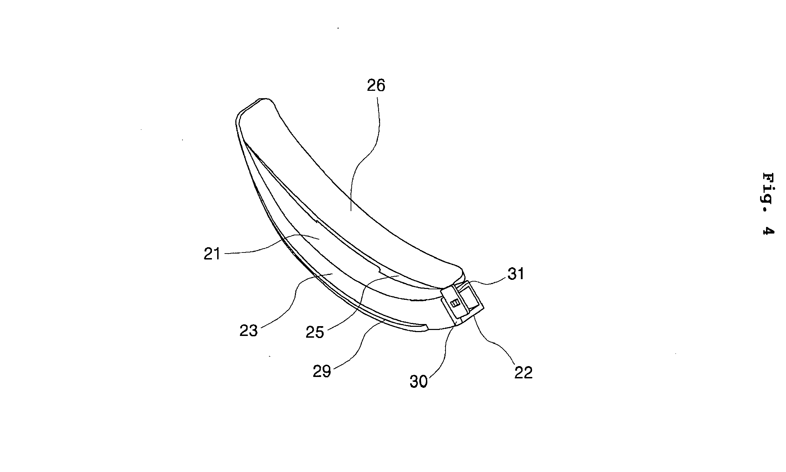

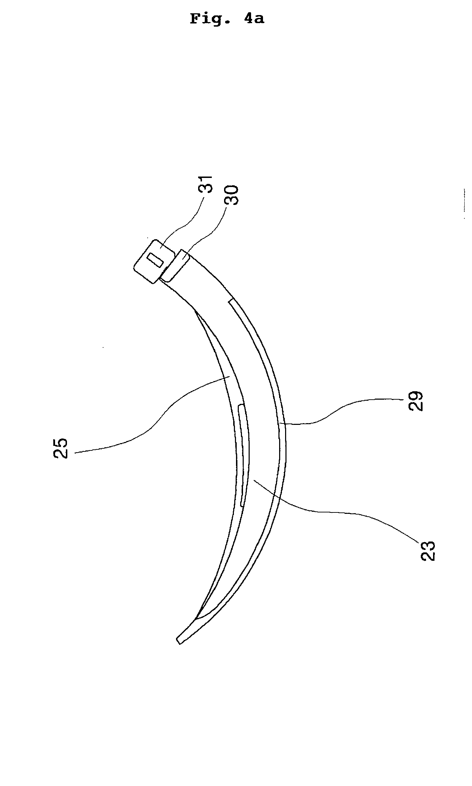

FIG. 1 is a perspective view illustrating a video laryngoscope according an embodiment of the present invention, FIG. 2 is an exploded perspective view illustrating a video laryngoscope according an embodiment of the present invention, and FIGS. 3 to 4 are views showing a blade according to an embodiment of the present invention.

Referring to the drawings, a video laryngoscope according to the present invention includes a grip part 40 provided therein with a body, a front end part 10 that forms a curved frame protruding from a lower end of the grip part 10 in which a camera lens is installed on an end of the frame, and a blade 20 fitted to the front end part 10.

The blade 20 includes a top surface 21 curved downward with a curvature identical to a curvature of the front end part 10, a bottom surface 22 curved downward to an extent of a middle part of the bottom surface 22 with a curvature identical to the curvature of the front end part 10, and first and second lateral sides 23 and 24 to connect the top and bottom surfaces 21 and 22 with each other.

A partition wall 25 is formed on the top surface 21 above the second lateral side 23 and the partition wall 25 is curved with a curvature less than the curvature of the front end part 10.

At an upper portion of the partition wall 25, a guide wing 26 extends in one direction to the front end part 10 with a curvature identical to a curvature of the partition wall 25 to form a guide space 28, which is curved downward in the same direction of the front end part 10, at one side of the front end part 10.

A slot having a curvature identical to the curvature of the front end part 10 is formed at one side of the bottom surface 22 and a soft wing 27 is detachably installed in the slot.

Therefore, according to the present invention, a height difference is caused due to the partition wall 25, so that the guide space 28 having a wide rear end and a narrow front end is defined at one side of the front end part 10 by the guide wing 26 curved at a curvature less than a curvature of the front end part 10 and a soft wing 27 curved at a curvature identical to a curvature of the front end part 10.

Therefore, a tube 1 can be easily inserted into the guide space 28 and can be easily handled.

In addition, a press wing 29 is provided at the bottom surface 22 of the blade 20. The press wing 29 is formed at one side of the bottom surface to facilitate the press operation for ensuring the free airway. The press wing 29 is curved with a curvature identical to a curvature of the bottom surface 22.

Further, a through hole is formed in the partition wall 25 to allow communication between the top surface 21 and the guide surface 28.

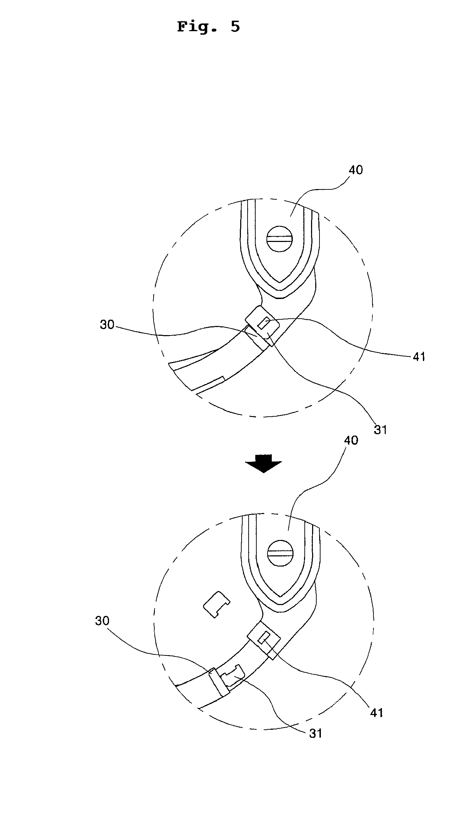

FIG. 5 is a side view showing states before and after the blade is fitted to the front end part.

Referring to FIG. 5, a protrusion 30 is provided at an edge of a rear end of the blade 20, and a plate 31 formed therein with a latch hole 32 protrudes rearward from the protrusion 30.

When the blade 20 is fitted to the front end part 10, the plate 31 is locked with a latch 41 provided at a lower end of the grip part so that the plate 31 is fixed.

Only one side end of the plate 31 is connected to the protrusion 30 about the latch hole 32, so, when the blade 20 is pulled after use, the plate 31 is divided about the latch hole 32 so that the blade 20 is separated from the front end part 10.

In this case, since the plate 31 for fixing the blade 20 to the front end part 10 is broken into two parts, the blade 20 cannot be reused.

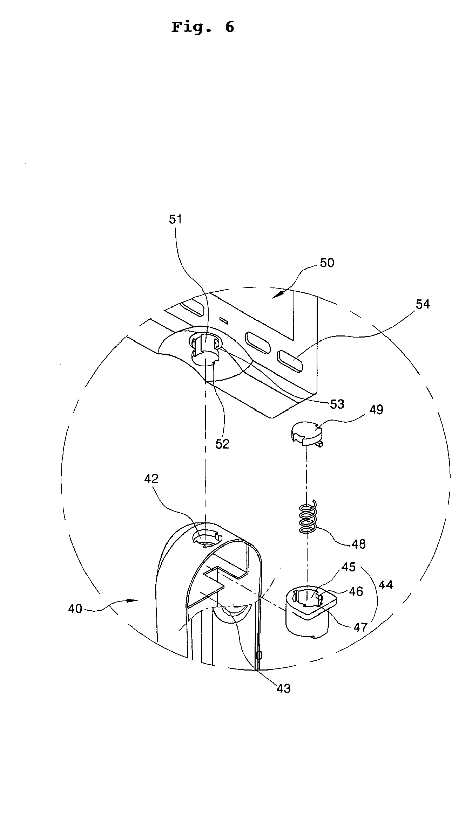

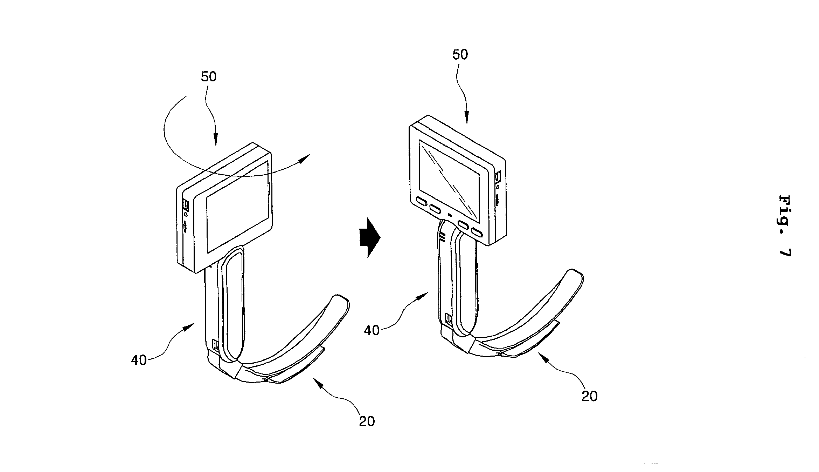

FIGS. 6 and 7 illustrate a coupling part formed in the grip part and a display device detachably coupled to the coupling part.

Referring to FIGS. 6 and 7, a cover member is detachably assembled to the grip part 40. The grip part 40 is provided therein with a body connected to the lens and a light mounted on the front end part 10. In addition, a battery is accommodated in the grip part 40 to supply power to the body.

A control button and a power button are provided at an outside of the grip part 40 to photograph an image of the larynx when the operator checks the larynx by gripping the grip part 40. In addition, the coupling part is provided at an upper end of the grip part 40 in order to mount a display device 50 thereon.

A coupling hole 42 perforated in a longitudinal direction is formed at an upper end of the grip part 40, and a support plate 43, which has a slot formed in a transverse direction to allow an assembling box to be fitted to the slot, is provided below the coupling hole 42.

The assembling box 44 is slidably assembled on the support plate 43.

The assembling box 44 has a receiving space 45 communicated with the coupling hole 42, a pair of guide plates 46 protrude from an upper edge of the receiving space 45 while facing each other, and a pair of guide grooves 47 are longitudinally formed in the receiving space 45.

In addition, a spring 48 is accommodated in the assembling box 44, and a cap 49 provided at both sides thereof with bars is mounted on the spring 48 above the receiving space. When the spring 48 positioned inside the assembling box 44 is compressed, the bars of the cap 49 move up and down along guide grooves 47 to open or close the coupling hole 42.

A cylindrical coupling rod 51 is assembled to a lower end of the display device 50, a pair of coupling holes 52 are formed at the edge of the coupling rod 51 in a longitudinal direction, and a recess 53, which is expanded radially outward, is formed in the coupling hole 52.

When the cap 49 of the assembling box 44 is pushed, the spring 48 is compressed. At this time, if the display device 50 is rotated, the guide plates 46 provided inside the receiving space 45 are locked at an inside of the recess 53 of the coupling rod 51 so that the display device 50 is coupled to the grip part 40.

In addition, if the display device 50 is rotated reversely, the guide plates 46 are separated out of the coupling grooves 52.

At this time, the separated display device 50 is placed on a bed or a chest of a patient, so the operator can check the state of the larynx and insert the tube 1 without changing the view point.

In addition, the display device 50 is detachably attached to the grip part and provided therein with a near field communication module and a battery in order to form a channel with respect to the body.

The near field communication module to form a wireless channel with respect to the body may include various types of communication modules, such as Bluetooth, WiFi, Zigbee and infrared communication modules, without limitation.

For reference, when the body provided inside the grip part 40 is powered on, the display device 50 is powered on.

When the control button of the body or the display device is manipulated, the state of the larynx is photographed through the image sensor and the lens provided in the front end part 10. In addition, photographed image information is transmitted to the display device through the wireless communication so that the state of the larynx may be displayed.

Therefore, according to the present invention, a height difference is caused due to the partition wall 25, so that the tube 1 can be readily inserted into the guide space 28 formed between the guide space 28 and the soft wing 27 and can be easily handled.

Therefore, a tube 1 can be easily inserted into the guide space 28 and can be easily handled.

In addition, since the plate 31 is broken into two parts when the blade 20 is separated from the front end part 10, the blade 20 cannot be reused.

Further, the operator can easily perform intubation of the tube 1 without changing the view point while checking the state of the larynx displayed on the display device 50 detachably coupled to the grip part 40.

## Claims

1. A video laryngoscope comprising:
a grip part provided therein with a body;
a front end part that forms a curved frame protruding from a lower end of the grip part in which an image sensor and a lens are installed on an end of the frame; and
a blade fitted to the front end part,
wherein the blade includes a top surface curved downward with a curvature identical to a curvature of the front end part, a bottom surface curved downward to an extent of a middle part of the bottom surface with a curvature identical to the curvature of the front end part, and first and second lateral sides to connect the top and bottom surfaces with each other,
a partition wall protrudes upward from the top surface with a curvature less than the curvature of the front end part,
a guide wing protrudes to one side from an upper portion of the partition wall while being connected to the front end part to form a guide space below the guide wing,
a slot having a curvature identical to the curvature of the front end part is formed at one side of the bottom surface and a soft wing is detachably installed in the slot, and
a tube is allowed to be readily inserted into the guide space formed between the guide wing and the soft wing such that the tube is easily handled.

2. The video laryngoscope of claim 1, further comprising a display device detachably assembled to an upper end of the grip part, wherein the display device is wirelessly connected to the body of the grip part and an image photographed by the front end part is displayed on the display device.

3. The video laryngoscope of claim 1, wherein a protrusion is provided at an edge of a rear end of the blade, a plate formed therein with a latch hole protrudes rearward from the protrusion, the plate is fixedly fitted to a latch formed at a lower end of the grip part when the blade is fitted to the front end part, and only one side end of the plate is connected to the protrusion about the latch hole, so, when the blade is pulled after use, the plate is divided about the latch hole so that the blade is separated from the front end part.

4. The video laryngoscope of claim 2, wherein a coupling hole perforated in a longitudinal direction is formed at an upper end of the grip part, a support plate, which has a slot formed in a transverse direction to allow an assembling box to be fitted to the slot, is provided below the coupling hole, and the assembling box is slidably assembled on the support plate.

5. The video laryngoscope of claim 4, wherein the assembling box slidably assembled on the support plate has a receiving space communicated with the coupling hole, a pair of guide plates protrude from an upper edge of the receiving space while facing each other, a pair of guide grooves are longitudinally formed in the receiving space, a spring is accommodated in the assembling box, and a cap provided at both sides thereof with bars is mounted on the spring above the receiving space, so, when the display device is attached or detached, the spring positioned inside the assembling box is compressed or expanded so that the cap moves up or down along the guide grooves to open or close the coupling hole.

6. The video laryngoscope of claim 5, wherein a coupling rod is provided at a lower end of the display device, a pair of coupling holes are formed in the coupling rod in a longitudinal direction, and a recess, which is expanded radially outward, is formed in the coupling hole.
